# EUROPEAN PATENT APPLICATION

(11) **EP 2 690 100 A1**
(43) Date of publication of application: **29.01.2014**
(21) Application number: 13190043.3
(22) Date of filing: 14.07.2011
(51) Int. Cl.: C07D 413/10

(54) **Process for making linezolid**

(30) Priority: 11.08.2010 WO PCT/EP2010/005209
(62) Divisional of application: 11733678.4
(71) Applicant: Synhton B.V., 6545 CM Nijmegen (NL)
(72) Inventor: Bartos, Petr, 67817 Blansko (CZ)
(74) Representative: Mendivil Gil, Maria Dolores

(57) **Abstract**

The present invention relates to a process for making linezolid comprising reacting in an aqueous medium a water soluble acid addition salt of the compound of formula (II) 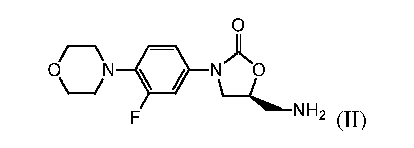 with acetyl halide or acetic acid anhydride in the presence of a base.

## Description

### BACKGROUND OF THE INVENTION

The present invention relates to an improved process for making the compound linezolid.

Linezolid is a pharmaceutically active compound useful as an antibacterial agent, e.g. for the treatment of diabetic food infections caused by Gram-positive bacteria. It is represented by the formula (I),

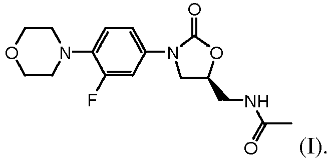

The marketed pharmaceutical compositions are a sterile isotonic solution for an i.v. infusion, a tablet for oral administration and an aqueous suspension for oral administration. They are marketed, i.e., under brand name ZYVOX by Pfizer.

The molecule of linezolid has one asymmetric carbon in the molecule allowing for 2 enantiomers; the marketed compound is the (S)-enantiomer. In the above-marketed compositions, linezolid is present as a free base.

Hereinunder, the name linezolid will be used as the generic name for N-(3-(3-fluoro-4-(morpholin-4-yl)phenyl)-2-oxooxazolidin-5(S)-ylmethyl)acetamide, unless indicated to the contrary.

Linezolid was first disclosed in WO 95/07271 (EP 0717738, US 5,688,792) of the Upjohn Company.

Various processes for making linezolid are known in the art. In particular, the important ones are these, the final step of which comprises acetylation of an amine precursor of the formula (II) with an acetylhalide or acetic anhydride (see, e.g., WO 2005 099353),

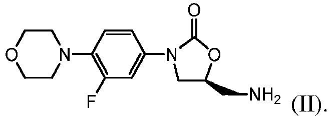

This amine precursor (II) may be made from various starting materials, e.g.:
a) By a reduction of an azide compound of formula (III) by a suitable reductant (WO2006/091731, WO 95/07271, US 5837870,WO2009/063505, US 7291614), 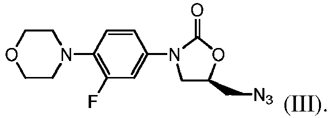 The starting compound (III) may be made from the corresponding tosylate or chloride of general formula (VII) below (WO 2005/099353).
b) By a decomposition of a phthalimide compound of formula (IV), e.g. by methylamine (WO95/07271) or by hydrazine (US 5837870), 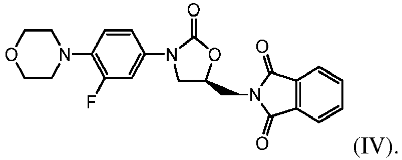 The starting compound (IV) may be made from the same tosylate or chloride as sub a) (WO2005/099353) or by a cyclization of the oxazolidine ring (WO 99/24393, WO2006/008754).
c) From a sulfonate compound of formula (V), 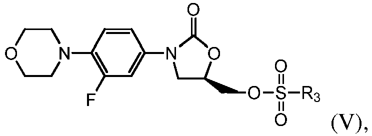 by treatment with ammonium hydroxide in isopropanol or THF (WO 95/07271) or by treatment with ammonia under enhanced pressure (WO 97/37980).
d) By a reduction of an imine (VI), 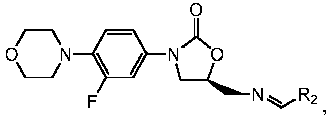 wherein R2 is a chlorophenyl, bromophenyl or 2,4,-dichlorophenyl moiety (WO 2007/116284).

Except of the imine (VI), each of the preceded synthetic approaches is based on a step of converting a starting material of the general formula (VII), 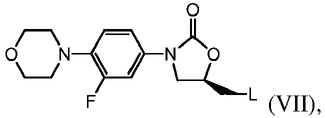 wherein L is a suitable leaving group, for instance a halogen or an alkyl-or aryl sulfonyloxy group,
by a reaction with a nitrogen nucleophile (an azide salt, phthalimide salt, ammonia or ammonium hydroxide), followed, if necessary, by a next step of conversion of the formed reaction intermediate (e.g., compound (III) or compound (IV)) into the amino/compound (II). Apparently, making the starting amine-compound (II) in a good yield and purity is the key aspect of commercial success of any of the above synthetic routes yielding linezolid. However, the known approaches have various drawbacks, for instance serious toxicity and explosion hazard of the azide salts, long reaction times and hazardous agents (hydrazine, methyl amine) in using the phthalimide intermediate, low yields and many side products at the ammonium hydroxide approach, or harsh reaction conditions in reaction with ammonia.

Thus, while many synthetic processes in making the compound (II) and the linezolid are known in the art, there still exists a need of an improvement. It would be desirable to have an alternate process of making the amine-intermediate of linezolid, which process is simple, runs at moderate reaction conditions and does not exhibit the above disadvantages.

### SUMMARY OF THE INVENTION

The present invention relates to a discovery of an improved process of making linezolid, which is simple in respect to process conditions and provides low amounts of reaction side products.

In a main aspect, the invention provides a process for making the compound 3-(3-fluoro-4-(morpholin-4-yl)phenyl)-2-oxooxazolidin-5(S)-ylmethyl)amine of formula (II) and/or an acid addition salt thereof, 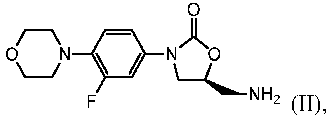 comprising
a) reacting the compound of formula (VII), 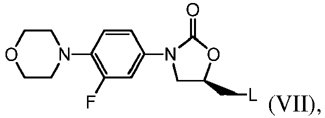 wherein L is a leaving group, with a metal salt of diformylamide of formula (VIII), 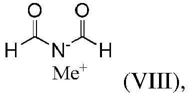 wherein Me⁺ is a sodium or potassium cation,
b) subjecting the reaction product of the step (a) comprising a mixture of compounds of formula (IXa) and (IXb), 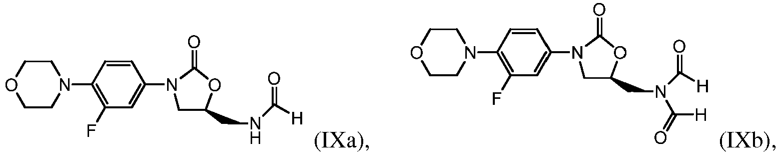 to a reaction with an acid, preferably with hydrochloric acid. The leaving group is preferably a halo group or an alkyl- or aryl-sulfonyloxy group. The compound (II) is converted to linezolid by acetylation, preferably as an acid addition salt and more preferably in water.

According to another aspect of the invention is provided a process for making linezolid comprising reacting in an aqueous medium a water soluble acid addition salt of the compound of formula (II), 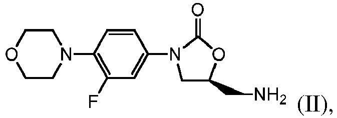 with acetyl halide or acetic acid anhydride in the presence of a base.

### DETAILED DESCRIPTION OF THE INVENTION

The starting material in the process of making linezolid according to the present invention is a compound of general formula (VII), 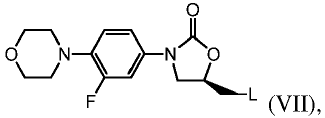 wherein L is a leaving group, typically a halo group or an alkyl- or aryl-sulfonyloxy group. The "halo" group comprises chloro- or bromo-group, preferably chloro-group. The "alkyl" comprises C1-C4 alkyl group and preferably a methyl group. The "aryl" comprises phenyl group, which may be optionally substituted by at least one C1-C4 alkyl group, a nitro-group, a hydroxyl group, a C1-C4 alkoxy group, and is preferably p-tolyl or p-nitrophenyl group.

The compounds are known in the art or may be prepared according to processes known in the art, e.g. from corresponding hydroxyl-methyl compounds of the formula (X),

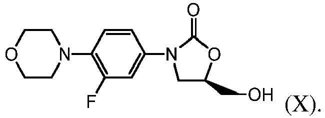

The second reaction partner is a metal salt of diformylamide of the formula (VIII),

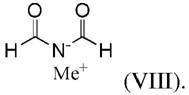

The "Me⁺" is typically sodium or potassium, preferably sodium. The compound (VIII) is known in the art and is commonly obtainable by a reaction of the corresponding metal methoxide with formamide, see US5488188 and US5599986. It may be isolated as a solid and stored at ambient conditions.

The reaction between the compounds (VII) and (VIII) may typically proceed in an inert solvent, which preferably is a polar aprotic solvent, for instance N,N-dimethyl formamide, dimethyl sulfoxide, acetonitrile, N-methylpyrrolidone, N,N-dimethylacetamide or acetone and mixtures thereof. Typically, molar ratio between compound (VII) and compound (VIII) is from 1:1 to 1:3, preferably of from about 1:1.1 to about 1: 1.7. The reaction temperature is typically from 50 to 100°C, preferably from 60 to 90°C. At these conditions, the reaction time is about 0.5 to 2 hours. The course of reaction may be monitored by common analytical techniques, e.g. by TLC or HPLC.

After the reaction is complete, the volatile residues are advantageously removed, e.g. by evaporation, and the residual metal salts are also removed, e.g. by an extraction with water.

The reaction product comprises a mixture of compounds of formula (IXa) and (IXb),

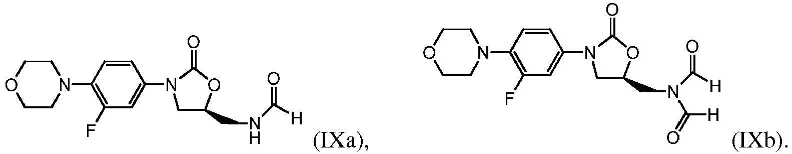

Mutual ratio of both compounds depends on the relative amount of the compound (VIII) in respect to the compound (VII). In borderline cases, the reaction mixture comprises only the compound (IXa) and/or only the compound (IXb). As both compounds (IXa) and (IXb) are equally convertible into the amine of the formula (II) in the next step, there is no objective need to separate them or enhance their mutual ratio in the mixture. However, it is not excluded that both compounds of the reaction mixture are separated by a suitable separation technique, for instance for analytical purposes. The compounds (IXa) and (IXb) may also be obtained in the form of an acid addition salt, e.g. as a hydrochloride, hydrobromide, sulphate, nitrate, phosphate, formate, acetate, propionate, oxalate, malonate, maleate, fumarate, citrate, malate etc.

Any of the compounds (IXa) and (IXb) and/or a mixture thereof is converted, in a next step, into the compound of formula (II) by a mild acid hydrolysis. Typically, the acid useful for this purpose is a strong mineral or organic acid, for instance hydrochloric acid, sulphuric acid, phosphoric acid,or hydrobromic acid. In general, a molar extent of the acid is used. The hydrolysis may proceed in a solvent, which may be water, a lower alcohol (C1-C4 aliphatic alcohol) or a mixture of both. Typical reaction temperature is between 0 and 80°C. The course of reaction may be monitored by a suitable analytical technique, e.g. HPLC or TLC.

The hydrolysis step may be advantageously combined with the preceded step in a one-pot arrangement.

As the rests of the reagents and solvents, as well as side products formed during the reaction are volatile compounds, the easiest way for removing them is evaporation, preferably at diminished pressure.

The product of the reaction comprises the raw amine compound of formula (II). It is formed after the hydrolysis as the corresponding acid addition salt (e.g. hydrochloride or dihydrochloride), which may be optionally neutralized into free amine, if necessary, by a treatment with an equivalent of a base, optionally followed by an extraction. The raw compound (II) and/or its acid addition salt, e.g. a hydrochloride or acetate may be, if necessary, purified by known processes and/or isolated in any solid state form, e.g. that as disclosed in US 2006/0258655. Hydrochloride or acetate salt of the compound (II) are the preferred salts.

Crude or purified amine (II) may be converted to linezolid by a reaction with an acetyl halide or acetic acid anhydride, under conditions known well in the art. In an advantageous way, the acetylation may be performed under conditions of Schotten-Baumann reaction or a modification thereof. The name "Schotten-Baumann reaction conditions" indicates the use of an acetylation agent in a presence of a base. Typically, the acetylation runs in a two-phase solvent system, consisting of water and a water immiscible organic solvent. A base is present within the water phase, which neutralizes the acid generated in the reaction, while the starting materials and product remain in the organic phase. Non-limiting examples of a water immiscible organic solvent are toluene, dichloromethane and/or diethyl ether.

In a suitable modification of the Schotten-Baumann reaction conditions, the compound (II) is converted, with or without isolation, into a water soluble acid addition salt of an inorganic acid or acetic acid, e.g. into a hydrochloride, hydrobromide, sulphate, nitrate, phosphate, and/or acetate. The salt is dissolved in an aqueous medium. Such aqueous medium is monophasic and comprises water and possibly water miscible solvents, such as alcohols, esters and the like, as long as the polarity is not changed such that the water soluble acid addition salts is not substantially dissolved in the aqueous medium and the linezolid formed substantially not. The aqueous medium, preferably water, is preferably cooled as close to 0°C as possible, the acetylation agent, preferably acetic anhydride, is added, and the acetylation process is started by continuous addition of a base (preferably an inorganic base, such as sodium carbonate or sodium acetate). The desired linezolid precipitates from the aqueous reaction mixture as it is not soluble therein. In this modification, organic solvents are completely avoided.

The produced linezolid may be isolated in any solid state form known in the art [Form I (J.Med.Chem. 39(3), 673 (1996)), Form II (WO 01/057035, US 6,559,305), Form III (WO 2005/035530) and many others (WO 2006/004922, US 2006/0142283), amorphous form (WO 2007/026369) and hydrated forms (US 2006/111350, EP 20033960)] and/or may be converted in a suitable acid addition salt.

The linezolid prepared by the process of the present invention can be formulated and used in pharmaceutical compositions. A suitable pharmaceutical composition may comprise linezolid and at least one pharmaceutically acceptable excipient. The compositions are useful as antibacterial agents, in treating various diseases caused by some types of bacteria, by administering an effective amount thereof to a patient in need of such treatment. In particular, they are useful in treatment of diabetic food infections caused by Gram-positive bacteria. Typically the effective amounts range from 1 mg to 500 mg, expressed as the amount of linezolid base, per day.

The invention will be further described with reference to the following non-limiting examples.

### EXAMPLES

### Example 1

### 3-(3-fluoro-4-(morpholin-4-yl)phenyl)-2-oxooxazolidin-5(S)-ylmethyl)amine (II) hydrochloride

To a 5 ml, reaction vial equipped with a magnetic stirring bar, 225 mg (0.5 mmol) of the compound (VII) [ L= p-toluenesulfonyloxy group] was added followed by 96 mg (1mmol) of sodium diformylamide and 2.5 ml of N,N-dimethylformamide. The vessel was immersed into an oil bath, which was preheated to 85°C and the mixture was stirred at external temperature of 85°C for 1 hour. The volatiles were evaporated at diminished temperature (30 mbar, 60°C), the residue was mixed with 10 ml of ethyl acetate and extracted with 10 ml of water. The organic layer was evaporated to dryness (45°C, 25 mbar) to yield a white solid.

The solid was mixed with 10 ml of methanol an 0.438 ml of concentrated hydrochloric acid (5.00 mmol) was added. The reaction mixture was warmed up in an oil bath to 65°C and stirred at this temperature for 2 hours. The mixture was evaporated at diminished pressure and then co-evaporated with toluene (60°C, 25 mbar) to yield 220 mg of white glassy solid.

### Example 2

### 3-(3-fluoro-4-(morpholin-4-yl)phenyl)-2-oxooxazolidin-5(S)-ylmethyl)amine (II)

A 250 ml, three-necked, round-bottomed flask equipped with a blade mechanical stirrer and Ar inlet combined with a temperature probe was placed under an argon atmosphere and charged with sodium diformylamide (5.6 g) and the compound (VII) [ L= p-toluenesulfonyloxy group] (20.8 g). N,N-Dimethylformamide (52 ml) was added and the reaction mixture was heated to 85°C for 2 h. Hydrochloric acid (98 ml) was carefully added into the reaction mixture, such that the internal temperature did not exceed 96°C. The heterogeneous reaction mixture was heated to 85°C and the reaction was continued for 2.5 h. The reaction mixture was transferred to a 500 ml round bottom flask and cooled down in an ice-water bath. Dichoromethane (150 ml) was charged to the flask. The mixture was vigorously stirred and a solution of NaOH (54.0 g) in water (100 ml) was added, such that the internal temperature did not exceed 20°C. The mixture was filtered. The filtrate was transferred to a 500 ml separatory funnel and the organic layer was separated. The filter cake was discarded. The water layer was extracted with dichloromethane (2 x 50 ml). The combined organic layer was dried over Na2SO4 (25 g), filtered, and concentrated (60°C, 50 mbar) to provide the product as a tan crystalline solid (18.87 g).

### Example 3

To a 25 ml, round-bottomed flask equipped with a magnetic stirring bar was charged "amine" (0.49 g) followed by water (8.30 ml). A heterogeneous mixture was stirred and hydrochloric acid (0.12 mL, 35 %) was added. A homogenous solution was obtained. The solution was cooled down in an ice-water bath to 0°C. Acetic anhydride (0.31 mL) was added followed by sodium bicarbonate (0.45 g). Carbon dioxide was immediately released and a formation of white precipitate was observed. The precipitate was filtered off and the filter cake was washed with water (10 ml). The filter cake was collected and dried (100 mbar) at 70°C overnight. An off-white solid linezolid (0.26 g) was isolated.

## Claims

1. A process for making linezolid comprising reacting in an aqueous medium a water soluble acid addition salt of the compound of formula (II) 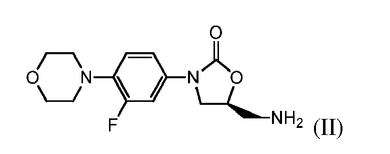 with acetyl halide or acetic acid anhydride in the presence of a base.

2. The process according to claim 11, wherein the water soluble acid addition salt of the compound of formula (II) is in a form of an acid addition salt with an inorganic acid or acetic acid.
